# NEW EUROPEAN PATENT SPECIFICATION

(11) **EP 0 470 795 B2**
(45) Date of publication and mention of the opposition decision: **21.02.2001**
(45) Mention of the grant of the patent: 25.01.1995
(21) Application number: 91307184.1
(22) Date of filing: 05.08.1991
(51) Int. Cl.: C07C 255/50, C07C 253/30, C07D 401/12, C07D 403/10

(54) **Process for the manufacture of nitriles**
Verfahren zur Herstellung von Nitrilen
Procédé pour la préparation de nitriles

(30) Priority: 09.08.1990 GB 9017482
(43) Date of publication of application: 12.02.1992
(73) Proprietor: Avecia Limited, Blackley, Manchester M9 8ZS (GB)
(72) Inventor: Roberts, David Anthony, Congleton, Cheshire CW12 2HH (GB); Russell, Simon Thomas, Macclesfield, Cheshire SK2 5HR (GB); Pittam, John David, Crewe, Cheshire CW4 8NU (GB)
(74) Representative: Revell, Christopher

(56) References cited:
- EP-A- 0 253 310
- EP-A- 0 324 377
- EP-A- 0 412 848
- WO-A-89/03821
- N. MIYAURA et al, Synthetic Communications, 11(7), 1981, pp. 513-519
- HOUBEN-WEYL, vol. 13/13a, Organoborverbindungen I, pp. 617, 832, 837

## Description

This invention concerns a novel process and, more particularly, it concerns a novel process for the manufacture of certain biphenylcarbonitriles which are useful, for example, as chemical intermediates in the production of certain known imidazole derivatives and certain novel quinoline derivatives which inhibit the action of angiotensin II (All) such derivatives being useful therefore in treating diseases or medical conditions such as hypertension or congestive heart failure. The invention further includes a novel process for the manufacture of said novel quinoline derivatives.

In European Patent Application, publication no. 253310 A2 (hereinafter EPA 253310) there is described the preparation of substituted imidazole derivatives useful as All inhibitors. Intermediates which are useful for the production of certain of these compounds include particular biphenylcarbonitriles. The preparation of one such biphenylcarbonitrile, 4'-methylbiphenyl-2-carbonitrile, is described in EPA 253310. This is obtained by a multi-step procedure which requires formation of 4'-methylbiphenyl-2-carboxylic acid, followed by stepwise funtionalisation of the carboxylic acid group. In EPA 253310 the starting acid is made, for example, by an Ullmann coupling reaction to obtain the corresponding ester which is then hydrolysed, or from hydrolysis of 2-(4'-methylbiphenyl-2-yl)-4,4-dimethyloxazoline itself obtained via a procedure involving several steps. In addition, the preparation of 4'-methyl-6-cyanobiphenyl-2-carbonitrile via 4-methylphenylzinc chloride is described in EPA 324377.

We have now discovered a convenient and useful alternative procedure for the production of 4'-methylbiphenylcarbonitriles.

According to the invention there is provided a process for the manufacture of a biphenylcarbonitrile of the formula I (set out hereinafter) wherein L¹ and L² are independently selected from hydrogen, (1-4C)alkyl, (1-4C)alkoxy, trifluoromethyl, cyano and nitro; which comprises reacting a boron compound of the formula II (set out hereinafter), wherein L¹ has any of the meanings defined hereinbefore and Q¹ and Q² are independently selected from hydroxy, (1-4C)alkoxy, (1-6C)alkyl and phenyl, the latter optionally substituted by a (1-4C)alkyl, (1-4C)alkoxy or halogeno group; or Q¹ together with Q² forms a (1-4C)alkylenedioxy group attached to the boron atom, a methylene group of which may optionally bear 1 or 2 (1-4C)alkyl groups; or Q¹ and Q² together with the boron atom to which they are attached form a boroxin ring of the formula IIa wherein L¹ has any of the meanings defined hereinbefore; with a compound of the formula III (set out hereinafter) wherein X¹ is a bromo or trifluoromethanesulphonyloxy group and L² has any of the meanings defined hereinbefore, in the presence of a suitable base and in the presence of a catalyst selected from a palladium(O), palladium(II), nickel(O) and nickel(II) catalyst: optionally in the presence of a radical initiator, and optionally in the presence ot lithium chloride.

It will be appreciated that generic terms such as "alkyl" include both straight and branched chain variants when the carbon numbers permit. However, when a particular radical such as "butyl" is given, it is specific to the straight chain variant. It will further be appreciated that the letter B used in formula II and IIa is the chemical symbol for boron.

Appropriate values for L¹ or L² include, by way of example, for alkyl: methyl and ethyl; for alkoxy: methoxy and ethoxy.

A particular value for Q¹ or Q² when it is alkyl is, for example, (1-4C)alkyl such as methyl, ethyl, propyl or butyl; and when it is alkoxy is, for example, methoxy or ethoxy.

A particular value for an optional substituent on Q¹ or Q² when it is phenyl is, for example, for alkyl: methyl or ethyl; for alkoxy: methoxy or ethoxy; and for halogeno: fluoro, chloro, bromo or iodo.

A particular value for Q¹ and Q² when together they form an alkylenedioxy in which a methylene group may optionally bear 1 or 2 alkyl groups is, for example, -O.CH₂.O-, -O.CH₂.CH₂.O., -O.CH₂.CH₂.CH₂.O- or -O.CH₂.C(CH₃)₂.CH₂.O-.

A preferred value for Q¹ and Q² is, for example, when they are both hydroxy or when Q¹ and Q² together with the boon atom to which they are attached form a boroxin ring of formula lla.

A preferred value for X¹ is bromo.

A preferred value for L¹ or L² is, for example, hydrogen.

Suitable catalysts include, for example, palladium(O), palladium(II), nickel(O) and nickel(II) catalysts, wherein the metal atom is attached to 4 groups independently selected from triphenylphosphine, triphenylphosphite, halogeno and acetyloxy, and palladium(II) halides and nickel(II) halides.

Particular catalysts include, for example, tetrakis(triphenylphosphine)nickel(O), bis(triphenylphosphine)-nickel(II) chloride, nickel(II)chloride, bis(triphenylphosphine)palladium(II) chloride, tetrakis-(triphenylphosphine)palladium(O) and palladium(II) chloride, of which the latter two are preferred, palladium-(II) chloride being particularly preferred.

A suitable base for use in the reaction is, for example, an alkali metal alkoxide such as sodium methoxide or sodium ethoxide, an alkali metal hydroxide such as sodium or potassium hydroxide, an alkali metal carbonate such as sodium or potassium carbonate, or an organic base such as a tri(1-6C)alkylamine, for example, triethylamine. A preferred base is an alkali metal carbonate, triethylamine or a mixture thereof.

A suitable radical initiator is, for example, azo(bisisobutyronitrile).

The process is generally performed in the presence of a suitable solvent or diluent, for example, a hydrocarbon, such as toluene or xylene, an ether, such as dioxan or tetrahydrofuran, an (1-4C)alcohol such as methanol, ethanol or butanol, water, or mixtures thereof. A preferred solvent is, for example, a mixture of water, toluene and methanol.

A preferred combination of catalyst, base and solvent for use in the process of the invention is, for example, tetrakis(triphenylphosphine)palladium(O), aqueous sodium carbonate and toluene, optionally in the presence of methanol, or palladium(II)chloride, aqueous sodium carbonate, toluene and methanol, optionally in the presence of triethylamine, the latter combination being particularly preferred.

The reaction is generally performed at a temperature in the range, for example, 50-150°C, and conveniently at or about the reflux temperature of the solvent or mixture of solvents used.

It will be seen that the process of the invention is significantly shorter than the procedures described in EPA 253310 for the preparation of 4'-methylbiphenyl-2-carbonitrile. It avoids the use of the Ullmann coupling reaction in the preparation of intermediates which generally requires particularly high temperatures and which is generally carried out with less readily available iodobenzenes. It also avoids the alternative procedure whereby formation of oxazoline compounds is required. In addition the starting materials required for the present invention are readily available, either commercially or by standard procedures of organic chemistry.

Compounds of the formula II as defined hereinbefore are known or may be obtained, for example, by reaction of a trialkylboronate of the formula **B(OR)**_{**3**} wherein R is a (1-6C)alkyl group with a Grignard reagent or phenyllithium compound derived, using standard procedures, from a compound of the formula IV wherein L¹ has any of the values defined hereinbefore and W is a halogeno group such as chloro, bromo or iodo. The reaction is generally carried out in a solvent such as tetrahydrofuran or ether, or a mixture thereof, and at a temperature in the range, for example, -78 °C to 25°C. Subsequent acid hydrolysis under standard conditions gives the boronic acids of formula II wherein Q¹ and Q² are both hydroxy groups, from which compounds of the formula II, wherein Q¹ and Q² together with the boron atom to which they are attached form a boroxin ring of the formula IIa as defined hereinbefore, may be obtained by dehydration using standard procedures. Alternatively said Grignard reagent or phenyllithium compound may be reacted with a borane of the formula **B(Q**^{**1**}**)(Q**^{**2**}**).Hal**. wherein Q¹ and Q² are alkyl or optionally substituted phenyl as defined above and **Hal**. is a halogeno group such as chloro, bromo or iodo. A compound of the formula II wherein Q¹ together with Q² forms an alkylenedioxy group attached to the boron atom may be obtained by reaction of a compound of the formula II wherein Q¹ and Q² are both hydroxy with an appropriate alkanediol (for example 2,2-dimethyl-propan-1,3-diol) in a suitable solvent such as cyclohexane at reflux with azeotropic removal of water.

In our co-pending EPA 412848 there is described a series of quinoline derivatives of formula V (set out hereinafter) wherein wherein R¹ is hydrogen, (1-8C)alkyl, (3-8C)cycloalkyl, phenyl or substituted (1-4C)alkyl, the latter containing one or more fluoro substituents or bearing a (3-8C)cycloalkyl, hydroxy, (1-4C)alkoxy or phenyl substituent; R² is hydrogen, (1-8C)alkyl, (3-8C)cycloalkyl, (3-8C)cycloalkyl-(1-4C)alkyl, carboxy, (1-4C)alkoxycarbonyl, cyano, nitro, phenyl or phenyl(1-4C)alkyl; R³ and R⁴ are independently selected from hydrogen, (1-4C)alkyl, (1-4C)alkoxy, fluoro(1-4C)alkoxy, halogeno, hydroxy, trifluoromethyl, cyano, nitro, amino, (1-4C)alkanoylamino, alkylamino and dialkylamino of up to 6 carbon atoms, dialkylamino-alkyl of 3 to 8 carbon atoms, (1-4C)alkanoyl, carbamoyl, N-alkylcarbamoyl and di-(N-alkyl)carbamoyl of up to 7 carbon atoms, carboxy, (1-4C)alkoxycarbonyl, (1-6C)alkylthio, (1-6C)alkylsulphinyl, (1-6C)alkylsulphonyl, and substituted (1-4C)alkyl, the latter bearing an amino, hydroxy or (1-4C)alkoxy substituent; or R³ and R⁴ together form (1-4C)alkylenedioxy attached to adjacent carbon atoms of the benzene moiety of formula V; Ra and R⁵ are independently selected from hydrogen, (1-4C)alkyl, (1-4C)alkoxy, halogeno, trifluoromethyl, cyano or nitro; A is methylene; X is phenylene optionally bearing a substituent selected from (1-4C)alkyl, (1-4C)-alkoxy, halogeno, trifluoromethyl, cyano and nitro, or X is a direct bond between the adjacent phenyl group and moiety A; Z is 1H-tetrazol-5-yl, -CO.NH.(1H-tetrazol-5-yl) or a group of the formula **-CO.OR**⁶ or **-CO.NH.SO**₂**.R**⁷in which R⁶ is hydrogen or a non-toxic, biodegradable residue of a physiologically acceptable alcohol or phenol, and R⁷ is (1-6C)alkyl, (3-8C)cycloalkyl or phenyl; and wherein any of said phenyl moieties may be unsubstituted or bear one or two substituents independently selected from (1-4C)-alkyl, (1-4C)alkoxy, halogeno, cyano and trifluoromethyl; or a non-toxic salt thereof; but excluding methyl 2-[(3-methoxycarbonylquinolin-4-yloxy)methyl]benzoate.

It will be appreciated that the compounds of formula V, depending on the nature of the substituents, may possess one or more chiral centres and may be isolated in one or more racemic or optically active forms.

In addition, in the compounds of formula V generic terms such as "alkyl" include both straight and branched chain variants when the carbon numbers permit. However, when a particular radical such as "propyl" is given, it is specific to the straight chain variant, branched chain variants such as "isopropyl" being specifically named where intended. The same convention applies to other radicals.

A particular value for R¹ or R² when it is alkyl is, for example, methyl, ethyl, propyl, butyl, isobutyl, sec-butyl, pentyl or hexyl; and when it is cycloalkyl is, for example, cyclopropyl, cyclopentyl or cyclohexyl.

A particular value for R¹ when it is alkyl bearing one or more fluoro substitutents is, for example, fluoromethyl, trifluoromethyl, 2,2,2-trifluoroethyl or pentafluoroethyl; and when it is alkyl bearing a hydroxy, cycloalkyl, (1-4C)alkoxy or phenyl substituent is, for example, hydroxymethyl, 1-hydroxyethyl, 2-hydroxyethyl, cyclopropylmethyl, cyclopentylmethyl, cyclohexylmethyl, 2-methoxyethyl, 2-ethoxyethyl, benzyl, 1-phenylethyl or 2-phenylethyl.

A particular value for R² when it is cycloalkyl-alkyl is, for example, cyclopropylmethyl, cyclopentylmethyl, cyclohexylmethyl or 2-cyclopentyl-ethyl; when it is phenylalkyl is, for example, benzyl, 1-phenylethyl or 2-phenylethyl; and when it is alkoxycarbonyl is, for example, methoxycarbonyl, ethoxycarbonyl or propoxycarbonyl.

Appropriate values for R³, R⁴, R⁵, or Ra, or for an optional substituent which may be present when X is phenylene, as defined above, include by way of example:-
for alkyl: methyl and ethyl; for alkoxy: methoxy and ethoxy; for fluoroalkoxy: trifluoromethoxy, 2-fluoroethoxy, 2,2,2-trifluoroethoxy and 3,3,3-trifluoropropoxy; for halogeno: fluoro, chloro, bromo and iodo; for alkanoylamino: formamido, acetamido and propanamido; for alkylamino: methylamino, ethylamino and butylamino; for dialkylamino: dimethylamino, diethylamino and dipropylamino; for dialkylamino-alkyl: dimethylaminomethyl, 2-(dimethylamino)ethyl, 2-(diethylamino)ethyl and 3-(diethylamino)propyl; for alkanoyl: formyl, acetyl and butyryl; for N-alkylcarbamoyl: N-methyl and N-ethylcarbamoyl; for di(N-alkyl)-carbamoyl: N,N-dimethylcarbamoyl and N,N-diethylcarbamoyl; for alkoxycarbonyl: methoxycarbonyl, ethoxycarbonyl and propoxycarbonyl; for alkylthio: methylthio, ethylthio and butylthio; for alkylsulphinyl: methylsulphinyl, ethylsulphinyl and butylsulphinyl; and for alkylsulphonyl: methylsulphonyl, ethylsulphonyl and butylsulphonyl; for alkyl bearing an amino, hydroxy or alkoxy substituent: hydroxymethyl, 1-hydroxyethyl, 2-hydroxyethyl, aminomethyl, 2-aminoethyl, 2-methoxyethyl and 2-ethoxyethyl; and alkylenedioxy: methylenedioxy and ethylenedioxy.

A particular value for R⁶ when it is a non-toxic, biodegradable residue of a physiologically acceptable alcohol or phenol is, for example, a residue derived from a (1-6C)alkanol such as methanol or ethanol, or phenol, glycerol or the like.

A particular value for R⁷ when it is alkyl is, for example, methyl, ethyl, propyl, isopropyl, butyl or pentyl; and when it is cycloalkyl is, for example, cyclobutyl, cyclopentyl or cyclohexyl.

Particular values for optional substituents which may be present on one or more phenyl moieties include, by way of example, for halogeno: fluoro, chloro and bromo; for alkyl: methyl and ethyl; and for alkoxy: methoxy and ethoxy.

A specific value for X which is of particular interest is, for example, p-phenylene.

A preferred value for R⁶ or R⁵ is, for example, hydrogen and for R¹ is, for example, methyl, ethyl or propyl.

A preferred group of compounds described in our co-pending application comprises those compounds of the formula Va (set out hereinafter) wherein R¹, R², R³, R⁴ and R⁵ have any of their meanings as defined above and Z¹ is carboxy, 1H-tetrazol-5-yl or benzenesulphonamido, the latter optionally containing one or two substituents independently selected from halogeno (such as fluoro, chloro or bromo), (1-4C)alkyl (such as methyl or ethyl), (1-4C)alkoxy (such as methoxy or ethoxy), cyano, nitro and trifluoromethyl; together with the non-toxic salts thereof.

A preferred value for Z or Z¹ is, for example, carboxy or 1H-tetrazol-5-yl, which latter is especially preferred and, in particular, when it is attached ortho to the group X.

A particularly preferred combination of values in any of the above definitions is wherein the quinoline moiety together with the attached substituents R¹, R², R³ and R⁴, and Ra when present, has any of the following values:- 2-methylquinoline, 2-ethylquinoline, 2-ethyl-6-methoxyquinoline, 6,7-dimethoxy-2-ethylquinoline, 2-ethyl-5,6,7-trimethoxyquinoline, 2-ethyl-6-hydroxyquinoline, 2-ethyl-6-methylthioquinoline, 2-ethyl-7-hydroxymethylquinoline, 2-ethyl-6-(2-fluoroethoxy)quinoline, 2-ethyl-6-(2,2,2-trifluoroethoxy)quinoline, 2-ethyl-6-carboxamidoquinoline, 2-ethyl-6-fluoroquinoline, 2-ethyl-6-isopropoxyquinoline or 6-aminomethyl-2-ethylquinoline; and in which the substituent O.A.X- is attached at the 4-position of the quinoline ring.

Compounds disclosed in our co-pending application which are particularly preferred are 2-methyl-4-[(2'-(1H-tetrazol-5-yl)biphenyl-4-yl)methoxy]quinoline, 2-ethyl-4-[(2'-(1H-tetrazol-5-yl)biphenyl-4-yl)methoxy]-quinoline, 2-ethyl-7-hydroxymethyl-4-[(2'-(1H-tetrazol-5-yl)biphenyl-4-yl)methoxy]quinoline, 2-ethyl-6-(2-fluoroethoxy)-4-[(2'-(1H-tetrazol-5-yl)biphenyl-4-yl)methoxy]quinoline, 2-ethyl-6-(2,2,2-trifluoroethoxy)-4-[(2'-(1H-tetrazol-5-yl)biphenyl-4-yl)methoxy]quinoline and 2-ethyl-6-isopropoxy-4-[(2'-(1H-tetrazol-5-yl)biphenyl-4-yl)methoxy]quinoline, together with their non-toxic salts.

Although all of the formula V compounds can form salts with suitable acids, it will be appreciated that those compounds of formula V wherein Z is other than an ester group or in which R³ or R⁴ is a carboxy group can form salts with acids as well as with bases. Particularly suitable non-toxic salts for such compounds therefore also include, for example, salts with bases affording physiologically acceptable cations, for example, alkali metal (such as sodium and potassium), alkaline earth metal (such as magnesium and calcium), aluminium and ammonium salts, as well as salts with suitable organic bases, such as with ethanolamine, methylamine, diethylamine or triethylamine, as well as salts with acids forming physiologically acceptable anions, such as salts with mineral acids, for example with hydrogen halides (such as hydrogen chloride and hydrogen bromide), sulphuric and phosphoric acid, and with strong organic acids, for example with p-toluenesulphonic and methanesulphonic acids.

The compounds of the formula V are described in EPA 412848 as obtainable by a variety of standard procedures of organic chemistry well known in the art for the production of structurally analogous compounds. The present invention provides an alternative process, by carrying out the additional subsequent steps (a)-(e) illustrated in Scheme 1, for the production of quinoline derivatives of the formula V wherein X is optionally substituted p-phenylene and Z is tetrazolyl, that is compounds of the formula VI. In the formula VI compounds, and the intermediates formed in steps (a)-(e), the variables R¹, R², R³, R⁴, Ra, L¹ and L² have any of the meanings defined hereinbefore.

With regard to steps (a)-(e):-
Step (a) may be carried out using a tri(1-6C)alkyltin or triphenyltin azide in the presence of a suitable solvent, for example toluene or xylene, and at a temperature in the range, for example, 50-150°C, and conveniently at the reflux temperature of the solvent employed, followed by treatment with a suitable acid, for example a mineral acid such as hydrochloric acid. The tri(1-6C)alkyltin or triphenyltin azide may be prepared from the correspondingly substituted tin halide (for example tributyltin chloride) and an alkali metal azide (for example sodium azide) in water under standard conditions.
Step(b) is carried out by a protecting group P being affixed to a nitrogen of the tetrazole ring of the intermediate formed in step (a). For example, where the protecting group is triphenylmethyl, this may be performed by reacting the product from step (a) with triphenylmethyl chloride in dichloromethane in the presence of a base such as triethylamine under standard conditions and at a temperature in the range, for example, 0-50 °C.
Step (c) is carried out by radical bromination using a halogenating agent such as N-bromosuccinimide in the presence of a radical initiator, such as azo(bisisobutyronitrile) or benzoyl peroxide, in the presence of a suitable solvent or diluent, for example a chlorinated solvent such as carbon tetrachloride, and at a temperature in the range, for example, 50-100°C.
Step (d) is carried out by alkylation of a compound of the formula VIII with the product of step (c). The reaction is generally performed, for example, in the presence of a suitable base such as an alkali metal alkoxide such as sodium methoxide or ethoxide, an alkali metal hydride such as sodium hydride or an alkali metal carbonate such as sodium or potassium carbonate, and in a suitable solvent or diluent, for example a (1-4C)alkanol such as methanol or ethanol when an alkali metal alkoxide is used, or in a polar solvent such as N,N-dimethylformamide or N-methylpyrrolidone. The reaction is usually performed at a temperature in the range, for example, 40-120°C.
Step (e) may be carried out using a variety of conditions dependent on the nature of the protecting group P. For example, when it is triphenylmethyl, the decomposition conditions include, for example, acid catalysed hydrolysis in a mineral acid (such as aqueous hydrochloric acid) conveniently in a solvent (such as aqueous dioxan, aqueous methanol or aqueous ethanol or a solvent used in step (d)), and at a temperature in the range, for example, 0-50°C, and conveniently at or about ambient temperature. It will be appreciated that the reagents used for carrying out steps (a)-(e) are only by way of example and other reagents, and protecting groups P other than triphenylmethyl, are envisaged which are well known alternatives in the field of organic chemistry for carrying out such steps. The compounds of the formula VIII are already known and the remainder can be made by analogy therewith using standard procedures of organic chemistry well known in the art, for example as described in standard works of heterocyclic chemistry such as that edited by Elderfield, or by the method in Org. Syn., Coll. Vol. III, p.374 and p. 593.

A further aspect of the invention provides a novel process, by carrying out the additional subsequent steps (a), (b) and (c), illustrated in Scheme 1 referred to hereinabove, followed by the additional steps (f) and (g) illustrated in Scheme 2, for the production of compounds of the formula VII, and pharmaceutically acceptable salts thereof, wherein **Alk.** is a (3-10C)alkyl group; X² is selected from hydrogen, fluoro, chloro, bromo, iodo, nitro, trifluoromethyl and cyano; and L¹ and L² have any of the meanings defined hereinabove. These compounds are known from EPA 253310 to be useful as All antagonists.

It will be appreciated that steps (a), (b) and (c) may be carried out as described above. With regard to steps (f) and (g):-
Step (f) is carried out by alkylation of a compound of the formula XIII, wherein the variables are as defined hereinabove, with the product of step (c). The reaction is generally performed, for example, in the presence of a suitable base such as an alkali metal alkoxide, such as sodium methoxide or sodium ethoxide, or an alkali metal hydride such as sodium hydride, in a suitable solvent or diluent such as a (1-4C)alkanol, for example methanol or ethanol, or in a polar solvent or diluent such as N,N-dimethylformamide, and at a temperature in the range, for example, 0-50°C. The compounds of the formula XIII may be obtained, for example, by the methods described in EPA 253310.
Step (g) may be carried out as for step (e) above.

The invention will now be illustrated by the following non-limiting Examples in which, unless otherwise stated:-
(i) concentrations and evaporations were carried out by rotary evaporation in vacuo;
(ii) operations were carried out at room temperature, that is in the range 18-26°C;
(iii) yields, where given, are intended for the assistance of the reader only and are not necessarily the maximum attainable by diligent process development;
(iv) ¹H NMR spectra were normally determined at 200 MHz in CDCl₃ using tetramethylsilane (TMS) as an internal standard, and are expressed as chemical shifts (delta values) in parts per million relative to TMS using conventional abbreviations for designation of major peaks: s, singlet; m, multplet; t, triplet; br, broad; d, doublet; and
(v) the term "1H-tetrazol-5-yl" stands for "1-H-1,2,3,4-tetrazol-5-yl".

### Example 1

4-Methylphenylboronic acid (1.62 g; 0.012 mole) was added to a solution of 2-bromobenzonitrile (1.82 g; 0.01 mole), tetrakis(triphenylphosphine)palladium(O) (0.35 g; 3 mole%) and 2M aqueous sodium carbonate solution (10 ml) in toluene (20 ml) and the mixture heated at 80°C for 6 hours. The mixture was allowed to cool and hydrogen peroxide (30 wt. % solution in water; 0.5 ml) was added. The mixture was stirred for 20 minutes and then extracted with ether and the extracts dried (MgSO₄). The solvent was removed by evaporation and the resultant oil purified by chromatography on silica eluting with 15% ethyl acetate/hexane to give 4'-methylbiphenyl-2-carbonitrile as a solid (1.65 g), m.p. 44-46°C; NMR(d₆-DMSO): 2.40 (s, 3H), 7.30(d, 2H), 7.35-7.55 (m, 4H), 7.60-7.65(m, 1H), 7.75(d, 1H).

### Example 2

2M Sodium carbonate solution (200 ml) was added to a stirred mixture of 4-methylphenylboronic acid (30 g), 2-bromobenzonitrile (36.4 g), palladium (II) chloride (0.4 g), methanol (200 ml) and toluene (200 ml) at 5°C. The temperature rose to approximately 20°C and a solid precipitated. The reaction mixture was then heated at reflux for 2 hours. The reaction mixture was allowed to cool and water (100 ml) was added, followed by diatomaceous earth (5 g). The mixture was stirred for 15 minutes, then filtered through diatomaceous earth. The organic phase of the filtrate was separated and washed with 2M sodium carbonate solution and then water. The organic phase was then filtered and the filtrate evaporated. The resultant solid was recrystallised from petroleum ether (b.p. 110-120°C) to give 4'-methylbiphenyl-2-carbonitrile (in 80% yield) identical to that obtained in Example 1.

### Example 3

2-butyl-4-chloro-5-hydroxymethyl-1-[(2'-(triphenylmethyl-2H-tetrazol-5-yl)biphenyl-4-yl)methyl]imidazole (A) (1.0 g) was added to a 7.5M solution of hydrogen chloride in dioxan (10 ml) and water (1 ml) and the mixture stirred for one hour at ambient temperature. Volatile material was removed by evaporation, excess sodium carbonate solution added to the residue, and the mixture washed with ether (2x 10 ml). The aqueous layer was acidified to pH3 with 2M hydrochloric acid and extracted with dichloromethane. The extracts were dried (MgSO₄) and the solvent removed by evaporation. The residual white foam was triturated with ether and the resultant solid collected by filtration to give 2-butyl-4-chloro-5-hydroxymethyl-1-[(2'-(1H-tetrazol-5-yl)biphenyl-4-yl)methyl]imidazole (0.37 g), m.p. 179-180°C; NMR(CDCI₃): 0.9(t, 3H), 1.2-1.4(m, 2H), 1.5-1.7(m, 2H), 2.6 (t, 2H), 4.5(s, 2H), 5.2(s, 2H), 6.95(d, 2H), 7.15(d, 2H), 7.35-7.55(m, 3H), 7.80-7.90(m, 1H).

The starting material (A) was obtained as follows:-
(i) A solution of 4'-methylbiphenyl-2-carbonitrile (0.67 g) and tributyltin azide (9.3 g) in toluene (20 ml) was heated under reflux for 48 hours. The cooled reaction mixture was acidified by saturation with hydrogen chloride gas and then cooled in an ice bath. The suspended solid was collected by filtration and triturated with toluene to give 5-[2-(4'-methylbiphenylyl)-2H-tetrazole (B), m.p. 149-150°C, in 90% yield; NMR(CDCI₃/d₆-DMSO): 2.25(s, 3H), 6.95(d, 2H), 7.10(d, 2H), 7.50-7.70(m, 4H).
(ii) Triphenylmethyl chloride (18.73 g) was added to a stirred solution of compound B in dichloromethane (150 ml) at ambient temperature. Triethylamine (10.2 ml) was then added and the mixture heated at reflux for 2.5 hours. The reaction mixture was allowed to cool, washed with water and dried (MgSO₄). The solvent was removed by evaporation to give 5-[2-(4'-methylbiphenylyl)]-2-triphenylmethyl-2H-tetrazole (C) (26.7 g), m.p. 166-168°C; NMR(CDCl₃): 2.25(s, 3H), 6.90-7.00(m, 10H), 7.20-7.45(m, 12H), 7.85-7.90(m, 1H).
(iii) A mixture of compound C (0.54 g), N-bromosuccinimide (0.20 g) and azo(bisisobutyronitrile (18 mg) in carbon tetrachloride (10 ml) was heated under reflux for 3 hours. Insoluble material was removed by filtration and the filtrate concentrated. The residue was dissolved in ethyl acetate, washed with water and dried (MgSO₄). The solvent was removed by evaporation and the residue was triturated with ether to give 5-[2-(4'-bromomethylbiphenylyl)]-2-triphenylmethyl-2H-tetrazole (D) as a white solid, in 92% yield, m.p. 136-138°C; NMR(CDCl₃): 4.4(s, 2H), 6.85-7.10(m, 10H), 7.20-7.45(m, 12H), 7.95-8.00(m, 1H).
(iv) Sodium methoxide(0.54 g) was added to a stirred solution of 2-butyl-4-chloro-5-hydroxymethylimidazole (1.87 g) in DMF (25 ml) and the mixture cooled to 5°C. Compound D was added with stirring and the reaction mixture allowed to stir at ambient temperature for 72 hours. The solvent was removed by evaporation, and the residue dissolved in ethyl acetate and washed with water. The organic phase was dried (MgSO₄), the solvent removed by evaporation, and the residue purified by chromatography on silica eluting with ethyl acetate/hexane (1:1 v/v). There was thus obtained 2-butyl-4-chloro-5-hydroxymethyl-1-[(2'-(triphenylmethyl-2H-tetrazol-5-yl)biphenyl-4-yl)methyl]imidazole (A) as a solid (1.8 g), m.p. 89-93°C; NMR(CDCl₃): 0.85(t, 3H), 1.25(sextet, 2H), 1.60-1.75(m, 2H), 2.50(t, 2H), 4.3(s, 2H), 5.0(s, 2H), 6.75(d, 2H), 6.90-7.00(m, 6H), 7.10(d, 2H), 7.20-7.50(m, 12H), 7.90-7.95(m, 1H).

### Example 4

A mixture of 2-methyl-4-[(2'-(2-triphenylmethyl-2H-tetrazol-5-yl)biphenyl-4-yl)methoxy]quinoline (A) (890 mg) and a 7.5M solution of hydrogen chloride dioxane (10 ml) and water (1 ml) was allowed to stand for 72 hours. Volatile material was removed by evaporation and the residue was triturated with ether (2 x 50 ml). The ether was decanted off and the solid residue crystallised from isopropanol to give **2-methyl-4-[(2'-(1H-tetrazol-5-yl)blphenyl-4-yl)methoxy]quinoline hydrochloride** (370 mg), as a white solid, m.p. 188-190°C; NMR (d₆-DMSO): 2.92(s,3H), 5.63(s,2H), 7.21(d,2H), 7.56-7.87(m,8H), 8.07(dt,1H), 8.28(dd,1H), 8.32-(dd,1H); mass spectrum [-ve FAB, DMSO/NBA]: 392 (M-H)⁻, 158; microanalysis found: C,66.0; H,4.6; N,15.5%; C₂₄H₁₉N₅O.HCl.O.5H₂O requires: C,65.7; H,4.8; N,16.0%.

The starting material (A) was obtained as follows:-

Sodium hydride (60% dispersion in mineral oil; 90 mg) was added to a stirred solution of 2-methyl-4-quinolone (obtained as described in Org. Syn., 1955, Coll. Vol. III, page 374 and page 593) (340 mg) in DMF (10 ml). The mixture was stirred until a solution of hydrogen had ceased and a solution of 5-[2-(4'-bromomethylbiphenylyl)]-2-triphenylmethyl-2H tetrazole (1.2 g) in DMF (5 ml) was added. The mixture was stirred for 16 hours. The solvent was removed by evaporation and the residue partitioned between water (20 ml) and dichloromethane (2 x 10 ml). The organic layer was washed with saturated sodium chloride solution (5 ml) and dried (MgSO₄). The solvent was removed by evaporation and the resultant oil was purified by flash chromatography, eluting with methanol/dichloromethane (1:99 v/v) to give 2-methyl-4-[2'-(2-triphenyl-methyl-2H-tetrazol-5-yl)biphenyl-4-ylmethoxy]quinoline (A) (890 mg) as a white solid m.p. 168-170 °C (dec.); NMR: 2.7(s,3H), 5.14(s,2H), 6.7(s,1H), 6.9(dd,6H), 7.15- 7.55(complex m,17H), 7.65(dt,1H), 7.95-(m,2H), 8.1(dd,1H).

### Example 5

Using an analogous procedure to that described in Example 4, but starting from 2-ethyl-4-[2'-(2-triphenylmethyl-2H-tetrazol-5-yl)biphenyl-4-ylmethoxy]quinoline there was obtained **2-ethyl-4-[(2'-(1H-tetrazol-5-yl)biphenyl-4-yl)methoxy]quinoline hydrochloride** (A), in 70% yield; m.p. 178.181°C (dec.); NMR:(d₆-DMSO): 1.48(t,3H), 3.22(q,2H), 5.68(s,2H), 7.23(d,2H), 7.5-7.8(m,7H), 7.83(t,1H), 8.08(t,1H), 8.32-(t,2H). The starting material A was obtained using a similar procedure to that described in Example 4, but using 2-ethyl-4-quinolone, itself obtained using a similar procedure to that described for 2-methyl-4-quinolone but starting from aniline and ethyl propionylacetate.

### Example 6

4-Methyl-3-nitro-phenyl boronic acid (obtained as described in J.A.C.S., 1932, 54, 4415) (3.0g) was added to a solution of 2-bromobenzonitrile (2.73 g) and tetrakis(triphenylphosphine)palladium(O) (0.525 g) in a mixture of 2M aqueous sodium carbonate (15 ml) and toluene (38 ml). The mixture was heated at 100°C for 16 hours, then allowed to cool. The mixture was extracted with ethyl acetate and the extracts washed with saturated sodium chloride solution and dried. The solvent was removed by evaporation and the resultant light brown solid recrystallised from ethyl acetate to give 4'-methyl-3'-nitrobiphenyl-2-carbonitrile as a solid (3.13 g), m.p. 155-156°C; NMR(CDCl₃): 2.68(s, 3H), 7.44-7.86(complex m, 6H), 8.14(d, 1H); microanalysis, found: C, 70.3; H, 4.0; N, 11.8%; C₁₄H₁₀N₂O₂ requires: C, 70.6; H, 4.2; N, 11.8%.

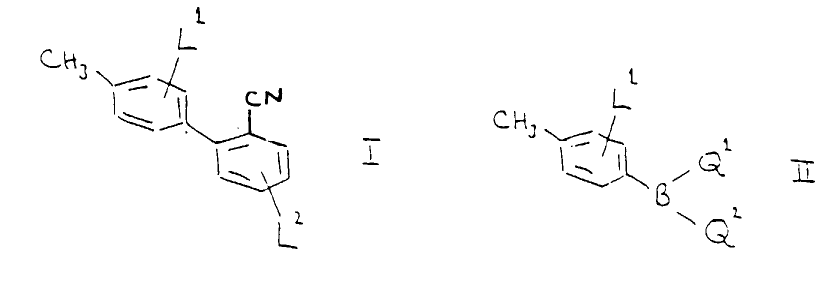

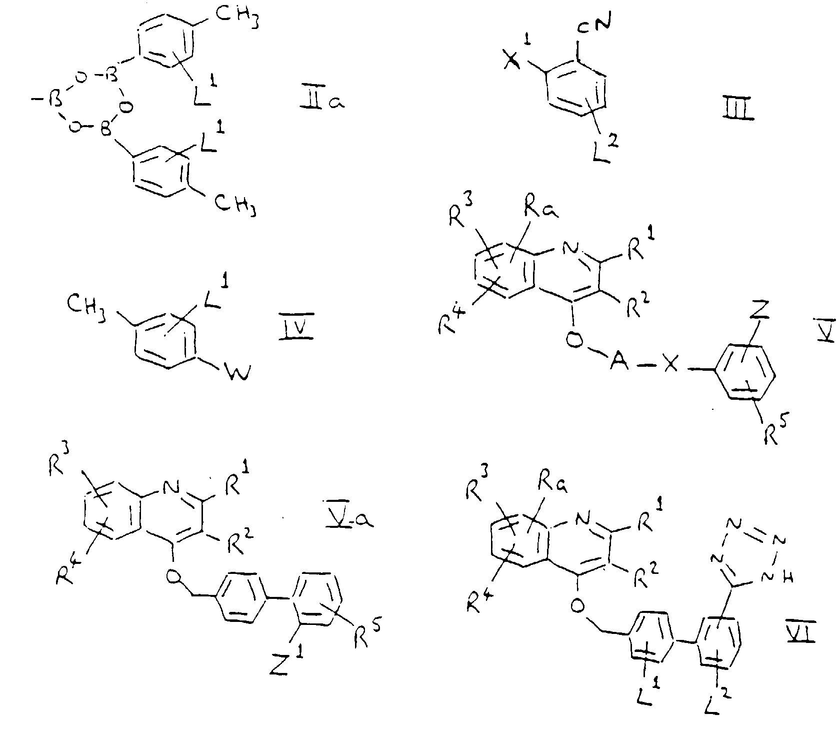

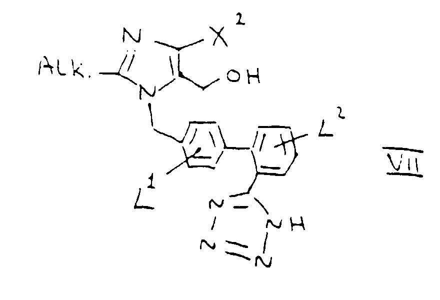

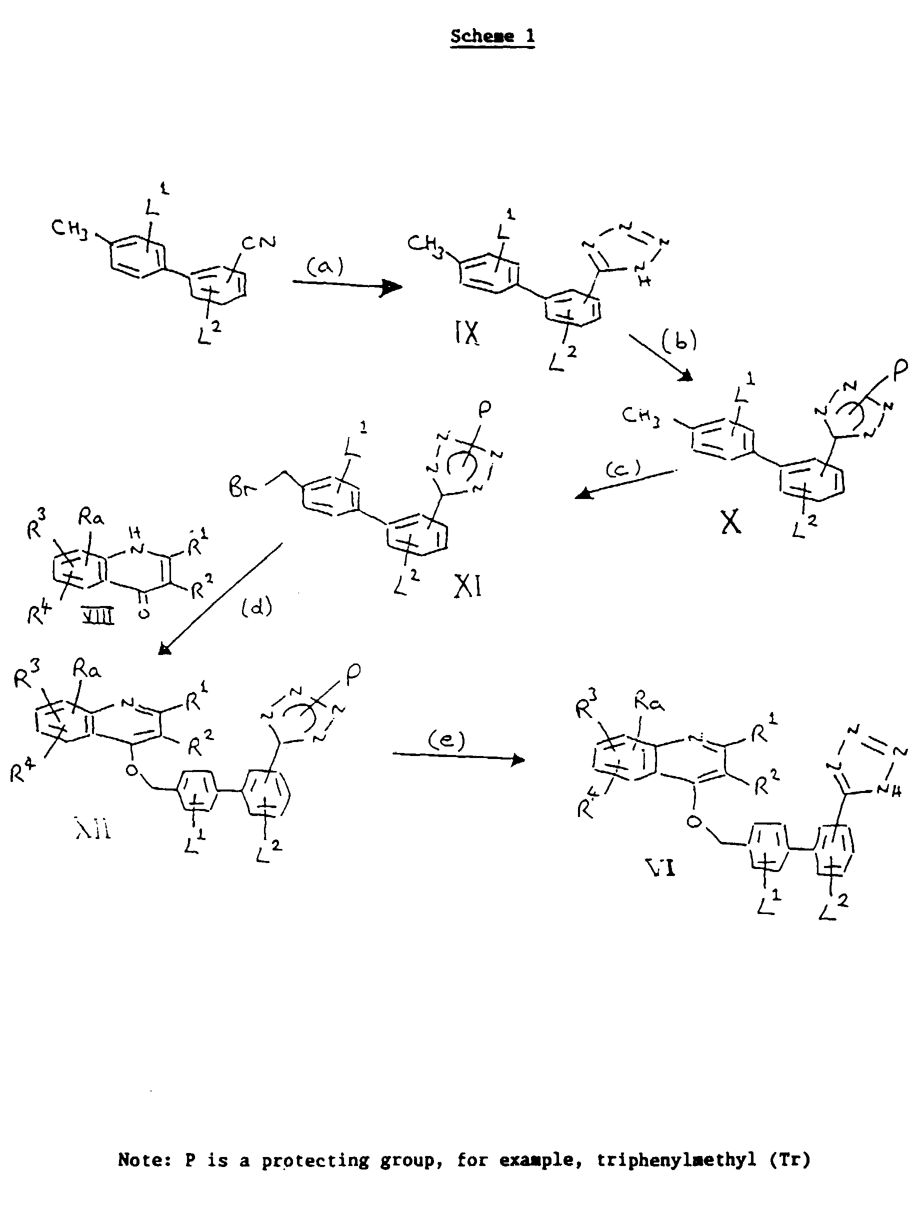

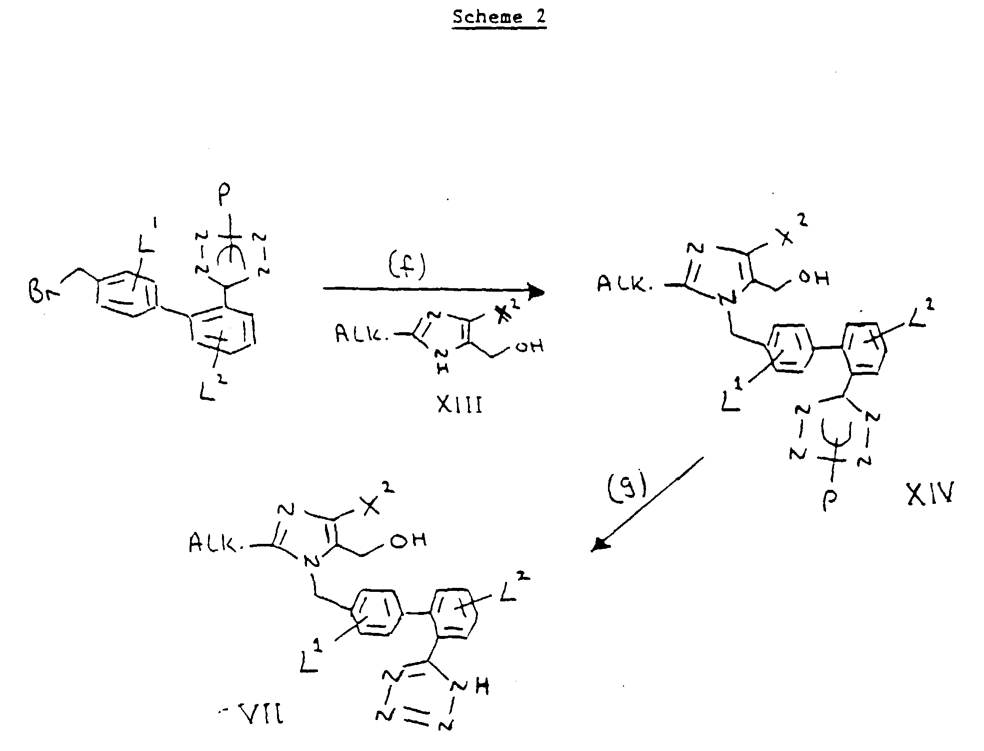

## Claims

1. A process for the manufacture of a biphenylcarbonitrile of the formula I 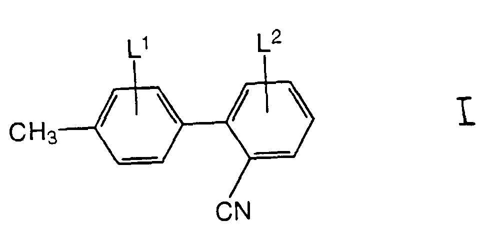 wherein L¹ and L² are independently selected from hydrogen, (1-4C)alkyl, (1-4C)alkoxy, trifluoromethyl, cyano and nitro; which comprises reacting a boron compound of the formula II 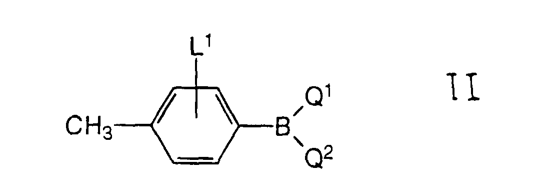 wherein L¹ has any of the meanings defined hereinbefore and Q¹ and Q² are independently selected from hydroxy, (1-4C)alkoxy, (1-6C)alkyl and phenyl, the latter optionally substituted by a (1-4C)alkyl, (1-4C)alkoxy or halogeno group; or Q¹ together with Q² forms a (1-4C)alkylenedioxy group attached to the boron atom, a methylene group of which may optionally bear 1 or 2 (1-4C)alkyl groups; or Q¹ and Q² together with the boron atom to which they are attached form a boroxin ring of the formula lla 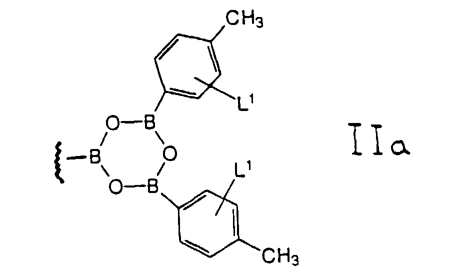 wherein L¹ has any of the meanings defined hereinbefore; with a compound of the formula III 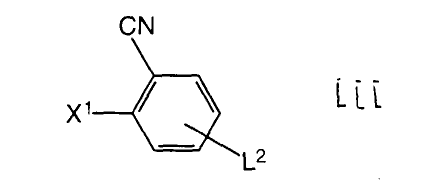 wherein X¹ is a bromo or trifluoromethanesulphonyloxy group and L² has any of the meanings defined hereinbefore, in the presence of a base and in the presence of a catalyst selected from a palladium(O), palladium(II), nickel(O) and nickel(II) catalyst; optionally in the presence of a radical initiator, and optionally in the presence of lithium chloride.

2. A process as claimed in claim 1 wherein, in the starting materials, L¹ and L² are independently selected from hydrogen, methyl, ethyl, methoxy, ethoxy, trifluoromethyl, cyano and nitro; Q¹ and Q² are independently selected from hydroxy, methoxy, ethoxy, methyl, ethyl, propyl, butyl and phenyl, the latter optionally substituted by a methyl, ethyl, methoxy, ethoxy, fluoro, chloro, bromo or iodo group; or Q¹ and Q² together with the boron atom to which they are attached form a boroxin ring of the formula lla wherein L¹ has any of the meanings defined hereinbefore; and wherein the catalyst is a palladium(O), palladium(II), nickel(O) or nickel(II) catalyst in which the palladium or nickel atom is attached to 4 groups independently selected from triphenylphosphine, triphenylphosphite, halogeno and acetyloxy, or is a palladium(II) halide or a nickel(II) halide.

3. A process as claimed in claim 1 or 2 wherein, in the starting materials, Q¹ and Q² are both hydroxy or Q¹ and Q² together with the boron atom to which they are attached form a boroxin ring of the formula IIa in which L¹ has any of the meanings defined in claim 1 or 2; X¹ is bromo; and the catalyst present is selected from tetrakis(triphenylphosphine)nickel(O), bis(triphenylphosphine)nickel(II) chloride, nickel(II) chloride, bis(triphenylphosphine)palladium(II)chloride, tetrakis(triphenylphosphine)palladium(O) and palladium(II) chloride.

4. A process as claimed in claim 1, 2 or 3 wherein the catalyst is tetrakis(triphenylphosphine)palladium(O) or palladium(II) chloride.

5. A process as claimed in any preceding claim wherein the base is an alkali metal carbonate. triethylamine or a mixture thereof.

6. A process as claimed in any preceding claim wherein a solvent, which is a mixture of water, toluene and methanol, is used.

7. A process for the manufacture of a compound of the formula VI 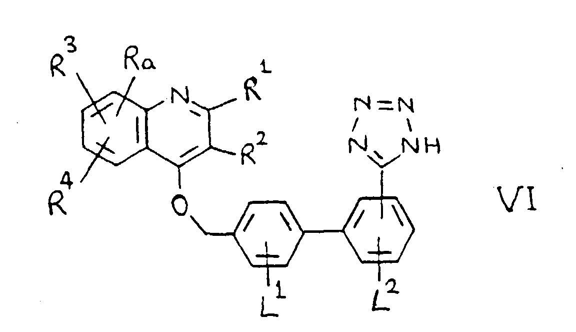 wherein R¹ is hydrogen, (1-8C)alkyl, (3-8C)cycloalkyl, phenyl or substituted (1-4C)alkyl, the latter containing one or more fluoro substituents or bearing a (3-8C)cycloalkyl, hydroxy, (1-4C)alkoxy or phenyl substituent; R² is hydrogen, (1-8C)alkyl, (3-8C)cycloalkyl, (3-8C)cycloalkyl-(1-4C)alkyl, carboxy, (1-4C)alkoxycarbonyl, cyano, nitro, phenyl or phenyl(1-4C)alkyl; R³ and R⁴ are independently selected from hydrogen, (1-4C)alkyl, (1-4C)alkoxy, fluoro(1-4C)alkoxy, halogeno, hydroxy, trifluoromethyl, cyano, nitro, amino, (1-4C)alkanoylamino, alkylamino and dialkylamino of up to 6 carbon atoms, dialkylamino-alkyl of 3 to 8 carbon atoms, (1-4C)alkanoyl, carbamoyl, N-alkylcarbamoyl and di-(N-alkyl)carbamoyl of up to 7 carbon atoms, carboxy, (1-4C)alkoxycarbonyl, (1-6C)alkyithio, (1-6C)alkylsulphinyl, (1-6C)-alkylsulphonyl, and substituted (1-4C)alkyl, the latter bearing an amino, hydroxy or (1-4C)alkoxy substituent; or R³ and R⁴ together form (1-4C)alkylenedioxy attached to adjacent carbon atoms of the benzene moiety of formula V; Ra is selected from hydrogen, (1-4C)alkyl, (1-4C)alkoxy, halogeno, trifluoromethyl, cyano or nitro; L¹ and L² have any of the values defined in claims 1 or 2; Z is 1H-tetrazol-5-yl; and wherein any of said phenyl moieties may be unsubstituted or bear one or two substituents independently selected from (1-4C)alkyl, (1-4C)alkoxy, halogeno, cyano and trifluoromethyl; or a non-toxic salt thereof; characterised by carrying out the following steps:-
(i) reacting a boron compound of the formula II with a compound of the formula III, wherein Q¹, Q² and X¹ have any of the meanings defined in claim 1, in the presence of a base and in the presence of a catalyst selected from a palladium(O), palladium(II), nickel(O) and nickel(II) catalyst: optionally in the presence of a radical initiator, and optionally in the presence of lithium chloride, to give a compound of the formula I;
(ii) reacting said compound of the formula I with a tri(1-6C)alkyl tin azide or triphenyltin azide in the presence of a solvent and then treatment with an acid to give a compound of the formula IX 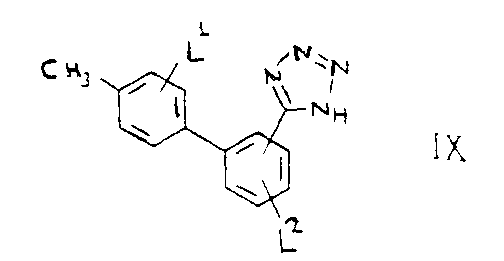
(iii) introducing a protecting group into the tetrazole ring of said compound of formula IX to give a compound of the formula X 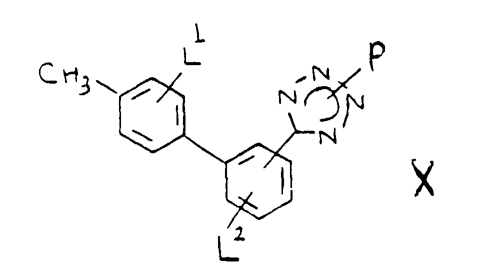 wherein P is a protecting group;
(iv) reacting said compound of formula X with a brominating agent in the presence of a radical initiator to give a compound of the formula XI 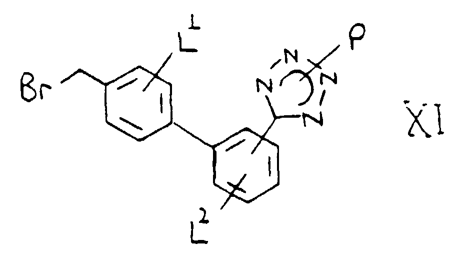
(v) alkylating a compound of the formula VIII 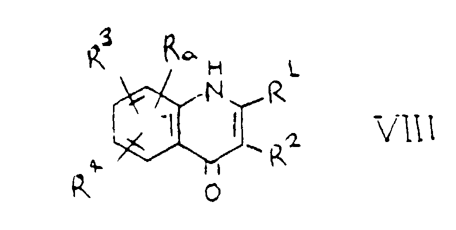 with said compound of formula XI to give a compound of formula XII 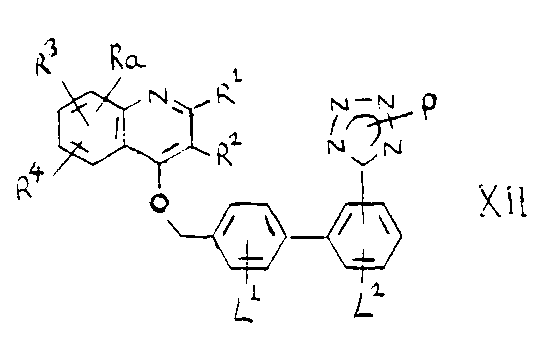 and
(vi) removing the protecting group P from said compound of formula XII to give said compound of formula VI;
whereafter: when a non-toxic salt of a compound of formula VI is required, it is obtained by reaction with the appropriate acid or base affording a physiologically acceptable ion, or by any other conventional salt formation procedure;
and wherein R¹, R², R³, R⁴, R⁵, Ra, L¹ and L² have any of the meanings defined above.

8. A process for the manufacture of a compound of the formula VII 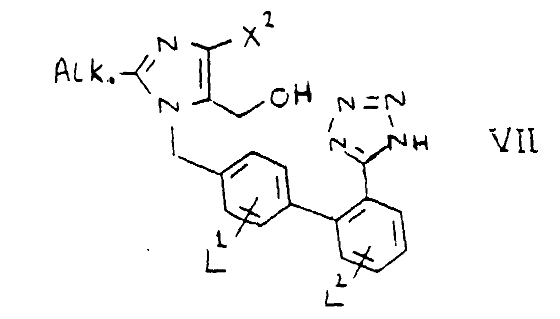 or a pharmaceutically acceptable salt thereof, wherein **Alk**. is a (3-10C)alkyl group; X² is selected from hydrogen, fluoro, chloro, bromo, iodo, nitro, trifluoromethyl and cyano; and L¹ and L² have any of the meanings defined in claim 1 or 2; characterised by carrying out steps (i), (ii), (iii) and (iv) as defined in claim 7 to give a compound of the formula XI, and then carrying out the following steps:-
(a) alkylating a compound of the formula XIII 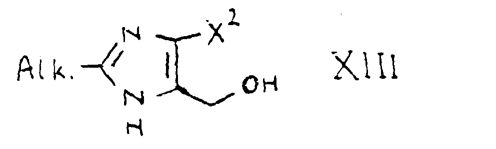 with said compound of formula Xl to give a compound of formula XIV 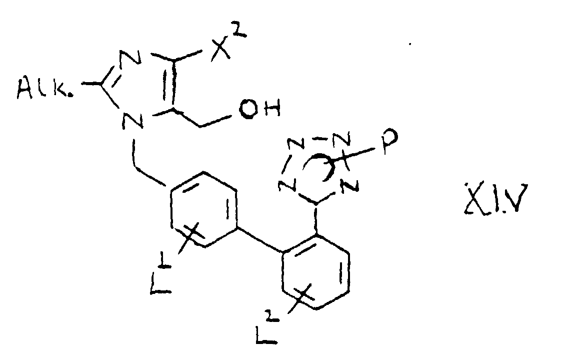 and
(b) removing the protecting group P from said compound of formula XIV to give said compound of formula VII;
whereafter: when a pharmaceutically acceptable salt of a compound of formula VII is required, it is obtained by reaction with the appropriate acid or base affording a physiologically acceptable ion, or by any other conventional salt formation procedure;
and wherein Alk., X², L¹ and L² have any of the meanings defined above.

## Patentansprüche

1. Verfahren zur Herstellung eines Biphenylcarbonitrils mit der folgenden Formel I 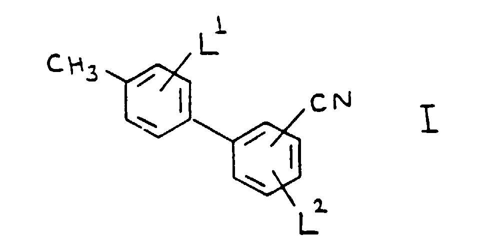 in der L¹ und L² unabhängig voneinander aus Wasserstoff, (1-4C)Alkyl, (1-4C)Alkoxy, Trifluormethyl, Cyano und Nitro ausgewählt sind;
bei dem eine Bor-Verbindung mit der folgenden Formel II 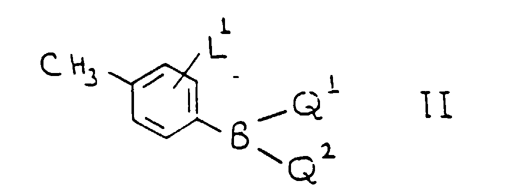 in der L¹ jede der oben definierten Bedeutungen hat und Q¹ und Q² unabhängig voneinander aus Wasserstoff, (1-4C)Alkoxy, (1-6C)Alkyl und Phenyl ausgewählt sind, wobei letzteres gegebenenfalls mit einer (1-4C)Alkyl-, (1-4C)Alkoxy- oder Halogen-Gruppe substituiert ist; oder in der Q¹ zusammen mit Q² eine (1-4C)Alkylendioxy-Gruppe bildet, die an das Boratom gebunden ist, wobei eine Methylen-Gruppe davon gegebenenfalls eine oder zwei (1-4C)Alkyl-Gruppen tragen kann; oder in der Q¹ und Q² zusammen mit dem Boratom, an das sie gebunden sind, einen Boroxin-Ring mit der folgenden Formel IIa bilden 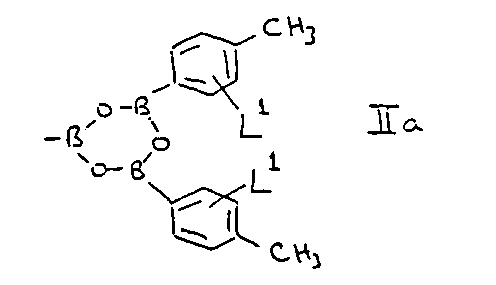 in der L¹ jede der oben definierten Bedeutungen hat; mit einer Verbindung mit der folgenden Formel III 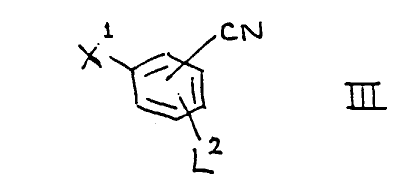 in der X¹ für eine Brom- oder Trifluormethansulfonyloxy-Gruppe steht und L² jede der oben definierten Bedeutungen hat, in Gegenwart einer Base und in Gegenwart eines Katalysators, der aus einem Palladium(O)-, Palladium(II)-, Nikkel(O)- und Nickel(II)-Katalysator ausgewählt ist, gegebenenfalls in Gegenwart eines Radikalinitiators und gegebenenfalls in Gegenwart von Lithiumchlorid umgesetzt wird.

2. Verfahren nach Anspruch 1, wobei in den Ausgangssubstanzen L¹ und L² unabhängig voneinander aus Wasserstoff, Methyl, Ethyl, Methoxy, Ethoxy, Trifluormethyl, Cyano und Nitro ausgewählt sind; Q¹ und Q² unabhängig voneinander aus Hydroxy, Methoxy, Ethoxy, Methyl, Ethyl, Propyl, Butyl und Phenyl ausgewählt sind, wobei letzteres gegebenenfalls mit einer Methyl-, Ethyl-, Methoxy-, Ethoxy-, Fluor-, Chlor-, Brom- oder Jod-Gruppe substituiert ist; oder wobei Q¹ und Q² zusammen mit dem Boratom, an das sie gebunden sind, einen Boroxin-Ring mit der Formel IIa bilden, in der L¹ jede der oben definierten Bedeutungen hat; und wobei es sich bei dem Katalysator um einen Palladium(O)-, Palladium(II)-, Nickel(O)- oder Nickel(II)-Katalysator handelt, in dem das Palladium- oder Nickelatom an 4 Gruppen gebunden ist, die unabhängig voneinander ausgewählt sind aus Triphenylphosphin, Triphenylphosphit, Halogen und Acetyloxy,oder bei dem es sich um ein Palladium(II)-Halogenid oder ein Nickel(II)-Halogenid handelt.

3. Verfahren nach Anspruch 1 oder 2, wobei in den Ausgangssubstanzen Q¹ und Q² beide für Hydroxy stehen, oder Q¹ und Q² zusammen mit dem Boratom, an das sie gebunden sind, einen Boroxin-Ring mit der Formel IIa bilden, in der L¹ jede der in Anspruch 1 oder 2 definierten Bedeutungen hat, X¹ für Brom steht und der vorhandene Katalysator ausgewählt ist aus Tetrakis(triphenylphosphin)nickel(O), Bis(triphenylphosphin)nickel(II)chlorid, Nickel(II)-chlorid, Bis(triphenylphosphin)palladium (II)chlorid, Tetrakis(triphenylphosphin)palladium(O) und Palladium(II)-chlorid.

4. Verfahren nach Anspruch 1, 2 oder 3, wobei es sich bei dem Katalysator um Tetrakis(trihenylphosphin)palladium(O) oder Palladium(II)-chlorid handelt.

5. Verfahren nach einem der vorhergehenden Ansprüche, wobei es sich bei der Base um ein Alkalimetallcarbonat, Triethylamin oder ein Gemisch daraus handelt.

6. Verfahren nach einem der vorhergehenden Ansprüche, wobei ein Lösungsmittel, bei dem es sich um ein Gemisch aus Wasser, Toluol und Methanol handelt, verwendet wird.

7. Verfahren zur Herstellung einer Verbindung mit der folgenden Formel VI 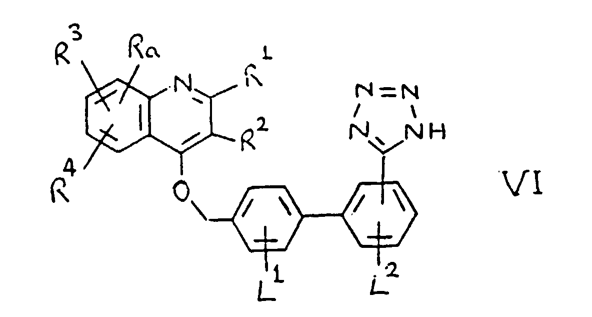 in der R¹ für Wasserstoff, (1-8C)Alkyl, (3-8C)Cycloalkyl, Phenyl oder substituiertes (1-4C)Alkyl steht, wobei letzteres einen oder mehrere Fluor-Substituenten enthält oder einen (3-8C)Cycloalkyl-, Hydroxy-, (1-4C)Alkoxy- oder Phenyl-Substituenten trägt; R² für Wasserstoff, (1-8C)Alkyl, (3-8C)Cycloalkyl, (3-8C)Cycloalkyl-(1-4C)alkyl, Carboxy, (1-4C)Alkoxycarbonyl, Cyano, Nitro, Phenyl oder Phenyl-(1-4C)alkyl steht; R³ und R⁴ unabhängig voneinander aus Wasserstoff, (1-4C)Alkyl, (1-4C)Alkoxy, Fluor(1-4C)Alkoxy, Halogen, Hydroxy, Trifluormethyl, Cyano, Nitro, Amino, (1-4C)Alkanoylamino, Alkylamino und Dialkylamino mit bis zu 6 Kohlenstoffatomen, Dialkylamino-alkyl mit 3 bis 8 Kohlenstoffatomen, (1-4C)Alkanoyl, Carbamoyl, N-Alkylcarbamoyl und Di-(N-alkyl)carbamoyl mit bis zu 7 Kohlenstoffatomen, Carboxy, (1-4C)Alkoxycarbonyl, (1-6C)Alkylthio, (1-6C)Alkylsulfinyl, (1-6C)Alkylsulfonyl und substituiertem (1-4C)Alkyl ausgewählt sind, wobei letzteres einen Amino-, Hydroxy- oder (1-4C)Alkoxy-Substituenten trägt; oder in der R³ und R⁴ zusammen (1-4C)Alkylendioxy bilden, das an benachbarte Kohlenstoffatome des Benzol-Restes der Formel V gebunden ist; Ra aus Wasserstoff, (1-4C)Alkyl, (1-4C)Alkoxy, Halogen, Trifluormethyl, Cyano oder Nitro ausgewählt ist; L¹ und L² jede der in den Ansprüchen 1 oder 2 definierten Werte haben; Z für 1H-Tetrazol-5-yl steht; wobei jeder der Phenyl-Reste unsubstituiert sein kann oder einen oder zwei Substituenten tragen kann, die unabhängig voneinander aus (1-4C)Alkyl, (1-4C)Alkoxy, Halogen, Cyano und Trifluormethyl ausgewählt sind; oder ein nichttoxisches Salz davon; dadurch gekennzeichnet, daß die folgenden Schritte durchgeführt werden:
(i) Umsetzung einer Bor-Verbindung mit der Formel II mit einer Verbindung mit der Formel III, wobei Q¹, Q² und X¹ jede der in Anspruch 1 definierten Bedeutungen haben, in Gegenwart einer Base und in Gegenwart eines Katalysators, der aus einem Palladium(O)-, Palladium(II)-, Nickel(O)- und Nikkel(II)-Katalysator ausgewählt ist, gegebenenfalls in Gegenwart eines Radikalinitiators und gegebenenfalls in Gegenwart von Lithiumchlorid, unter Erhalt einer Verbindung mit der Formel I;
(ii) Umsetzung der Verbindung mit der Formel I mit einem Tri(1-6C)alkylzinnazid oder Triphenylzinnazid in Gegenwart eines Lösungsmittels und dann Behandlung mit einer Säure unter Erhalt einer Verbindung mit der folgenden Formel IX 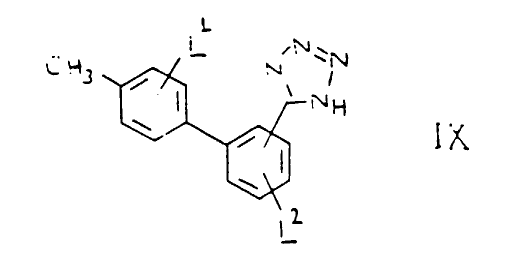
(iii) Einführung einer Schutzgruppe in den Tetrazol-Ring der Verbindung mit der Formel IX unter Erhalt einer Verbindung mit der folgenden Formel X 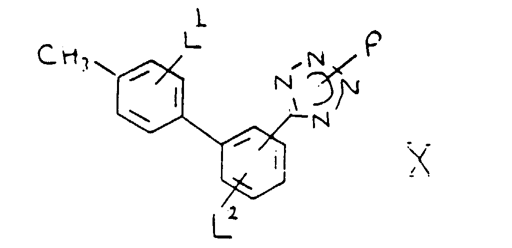 in der P für eine Schutzgruppe steht;
(iv) Umsetzung der Verbindung mit der Formel X mit einem Bromierungsmittel in Gegenwart eines Radikalinitiators unter Erhalt einer Verbindung mit der folgenden Formel XI 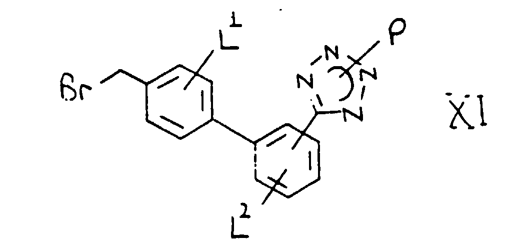
(v) Alkylierung einer Verbindung mit der folgenden Formel VIII 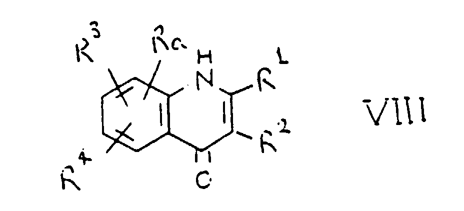 mit der Verbindung mit der Formel XI unter Erhalt einer Verbindung mit der folgenden Formel XII 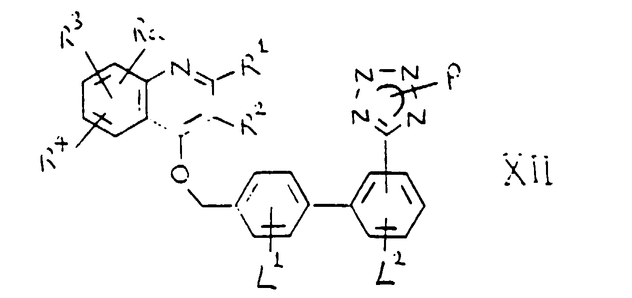 und
(vi) Entfernung der Schutzgruppe P aus der Verbindung mit der Formel XII unter Erhalt der Verbindung mit der Formel VI;
wonach, wenn ein nichttoxisches Salz einer Verbindung mit der Formel VI benötigt wird, dieses durch Umsetzung mit der geeigneten Säure oder Base erhalten wird, die ein physiologisch geeignetes Ion liefert, oder mit jedem anderen üblichen Salzbildungsverfahren; wobei R¹, R², R³, R⁴, R⁵, Ra und L¹' und L² jede der oben definierten Bedeutungen haben.

8. Verfahren zur Herstellung einer Verbindung mit der folgenden Formel VII 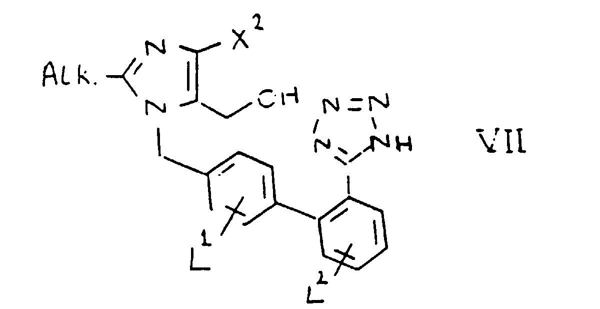 oder eines pharmazeutisch geeigneten Salzes davon, in der Alk. für eine (3-10C)Alkyl-Gruppe steht, X² aus Wasserstoff, Fluor, Chlor, Brom, Jod, Nitro, Trifluormethyl und Cyano ausgewählt ist und L¹ und L² jede der in Anspruch 1 oder 2 definierten Bedeutungen haben, dadurch gekennzeichnet, daß die in Anspruch 7 definierten Schritte (i), (ii), (iii) und (iv) unter Erhalt einer Verbindung mit der Formel XI durchgeführt werden und dann die folgenden Schritte durchgeführt werden:-
(a) Alkylierung einer Verbindung mit der folgenden Formel XIII 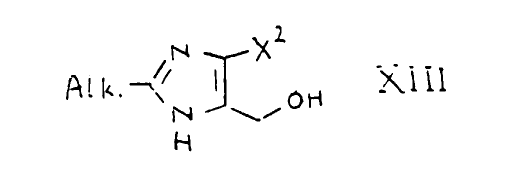 mit der Verbindung mit der Formel XI unter Erhalt einer Verbindung mit der folgenden Formel XIV 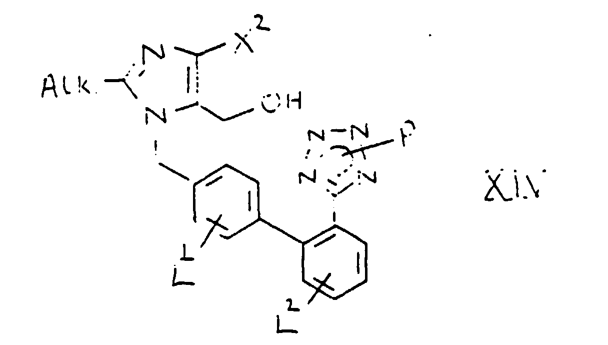 und
(b) Entfernung der Schutzgruppe P aus der Verbindung mit der Formel XIV unter Erhalt der Verbindung mit der Formel VII;
wonach, wenn ein pharmazeutisch geeignetes Salz einer Verbindung mit der Formel VII benötigt wird, dieses durch Umsetzung mit der geeigneten Säure oder Base erhalten wird, die ein physiologisch geeignetes Ion liefert, oder mit jedem anderen üblichen Salzbildungsverfahren, wobei Alk., X², L¹ und L² jede der oben definierten Bedeutungen haben.

## Revendications

1. Procédé de production d'un biphénylcarbonitrile de formule I 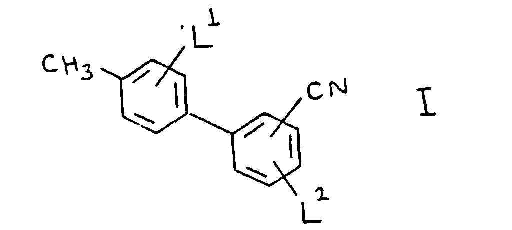 dans laquelle L¹ et L² sont choisis indépendamment entre l'hydrogène et des groupes alkyle en C₁ à C₄, alkoxy en C₁ à C₄, trifluorométhyle, cyano et nitro ; qui comprend la réaction d'un composé de bore de formule II 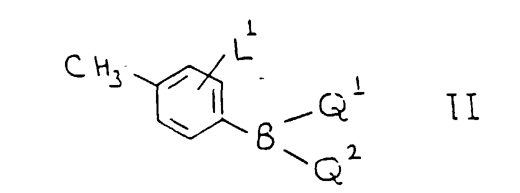 dans laquelle L¹ répond à l'une quelconque des définitions précitées et Q¹ et Q² sont choisis indépendamment entre des groupes hydroxy, alkoxy en C₁ à C₄, alkyle en C₁ à C₆ et phényle, ce dernier étant facultativement substitué avec un groupe alkyle en C₁ à C₄, alkoxy en C₁ à C₄ ou halogéno ; ou bien Q¹ en association avec Q² forme un groupe (alkylène en C₁ à C₄)-dioxy fixé à l'atome de bore, dont un groupe méthylène peut porter facultativement un ou deux groupes alkyle en C₁ à C₄ ; ou bien Q¹ et Q², conjointement avec l'atome de bore auquel ils sont fixés, forment un noyau boroxine de formule IIa 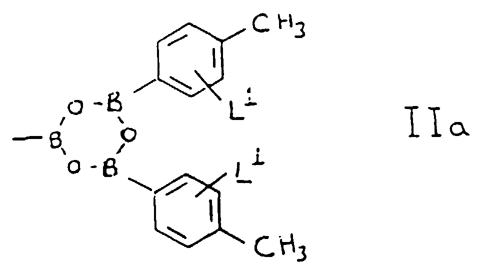 dans laquelle L¹ répond à l'une quelconque des définitions précitées ; avec un composé de formule III 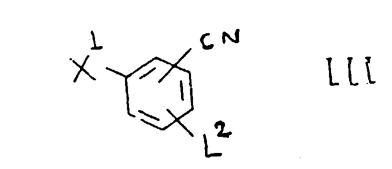 dans laquelle X¹ représente un groupe bromo ou trifluorométhanesulfonyloxy et L² répond à l'une quelconque des définitions précitées, en présence d'une base et en présence d'un catalyseur choisi entre un catalyseur au palladium(0), un catalyseur au palladium(II), un catalyseur au nickel(0) et un catalyseur au nickel(II) ; facultativement en présence d'un initiateur radicalaire, et facultativement en présence de chlorure de lithium.

2. Procédé suivant la revendication 1, dans lequel, dans les matières de départ, L¹ et L² sont choisis indépendamment entre l'hydrogène, les groupes méthyle, éthyle, méthoxy, éthoxy, trifluorométhyle, cyano et nitro ; Q¹ et Q² sont choisis indépendamment entre les groupes hydroxy, méthoxy, éthoxy, méthyle, éthyle, propyle, butyle et phényle, ce dernier étant facultativement substitué avec un groupe méthyle, éthyle, méthoxy, éthoxy, fluoro, chloro, bromo ou iodo ; ou bien Q¹ et Q², conjointement avec l'atome de bore auquel ils sont fixés, forment un noyau boroxine de formule IIa dans laquelle L¹ répond à l'une quelconque des définitions précitées ; et le catalyseur consiste en un catalyseur au palladium(0), un catalyseur au palladium(II), un catalyseur au nickel(0) ou un catalyseur au nickel(II) dans lequel l'atome de palladium ou de nickel est fixé à quatre groupes choisis indépendamment entre les groupes triphénylphosphine, triphénylphosphite, halogéno et acétyloxy, ou bien consiste en un halogénure de palladium(II) et un halogénure de nickel(II).

3. Procédé suivant la revendication 1 ou 2, danse lequel, dans les matières de départ, Q¹ et Q² représentent l'un et l'autre un groupe hydroxy ou bien Q¹ et Q², conjointement avec l'atome de bore auquel ils sont fixés, forment un noyau boroxine de formule IIa dans laquelle L¹ répond à l'une quelconque des définitions mentionnées dans la revendication 1 ou 2 ; ; X¹ représente un groupe bromo ; et le catalyseur présent est choisi entre le tétrakis(triphénylphosphine)nickel(0), le chlorure de bis(triphénylphosphine)nickel(II), le chlorure de nickel(II), le chlorure de bis (triphénylphosphine)palladium(II), le tétrakis(triphénylphosphine)palladium(0) et le chlorure de palladium(II).

4. Procédé suivant la revendication 1, 2 ou 3, dans lequel le catalyseur est le tétrakis(triphénylphosphine)palladium(0) ou le chlorure de palladium(II).

5. Procédé suivant l'une quelconque des revendications précédentes, dans lequel la base est un carbonate de métal alcalin, la triéthylamine ou un de leurs mélanges.

6. Procédé suivant l'une quelconque des revendications précédentes, dans lequel un solvant, qui est un mélange d'eau, de toluène et de méthanol, est utilisé.

7. Procédé pour la production d'un composé de formule VI 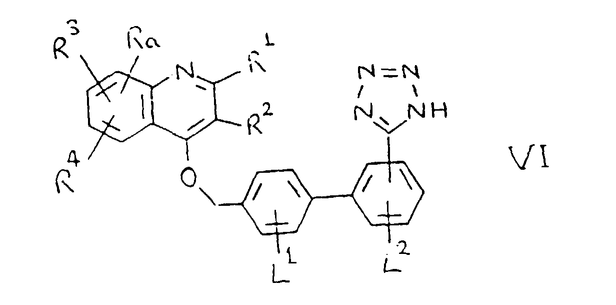 dans laquelle R¹ représente l'hydrogène, un groupe alkyle en C₁ à C₈, cycloalkyle en C₃ à C₈, phényle ou alkyle en C₁ à C₄ substitué, ce dernier contenant un ou plusieurs substituants fluoro ou bien portant un substituant cycloalkyle en C₃ à C₈, hydroxy, alkoxy en C₁ à C₄ ou phényle ; R² représente l'hydrogène, un groupe alkyle en C₁ à C₈, cycloalkyle en C₂ à C₈, (cycloalkyle en C₃ à C₈)-(alkyle en C₁ à C₄), carboxy, (alkoxy en C₁ à C₄)-carbonyle, cyano, nitro, phényle ou phényl-(alkyle en C₁ à C₄) ; R³ et R⁴ sont choisis indépendamment entre l'hydrogène, des groupes alkyle en C₁ à C₄, alkoxy en C₁ à C₄, fluoralkoxy en C₁ à C₄, halogéno, hydroxy, trifluorométhyle, cyano, nitro, amino, alcanoylamino en C₁ à C₄, alkylamino et dialkylamino ayant jusqu'à 6 atomes de carbone, dialkylaminoalkyle ayant 3 à 8 atomes de carbone, alcanoyle en C₁ à C₄, carbamoyle, N-alkylcarbamoyle et di-N- alkylcarbamoyle ayant jusqu'à 7 atomes de carbone, carboxy, (alkoxy en C₁ à C₄)-carbonyle, alkylthio en C₁ à C₆, alkylsulfinyle en C₁ à C₆, alkylsulfonyle en C₁ à C₆ et alkyle en C₁ à C₄ substitué, ce dernier portant un substituant amino, hydroxy ou alkoxy en C₁ à C₄ ; ou bien R³ et R⁴ forment conjointement un groupe alkylènedioxy en C₁ à C₄ fixé à des atomes de carbone adjacents du groupement benzénique de formule V ; Ra est choisi entre l'hydrogène et un groupe alkyle en C₁ à C₄, alkoxy en C₁ à C₄, halogéno, trifluorométhyle, cyano ou nitro ; L¹ et L² possèdent n'importe lesquelles des valeurs définies dans la revendication 1 ou 2 ; Z représente un groupe 1H-tétrazole-5-yle ; et n'importe lequel desdits groupements phényle peut être non substitué ou bien peut porter un ou deux substituants choisis indépendamment entre des groupes alkyle en C₁ à C₄, alkoxy en C₁ à C₄, halogéno, cyano, trifluorométhyle ; ou d'un de ses sels non toxiques ; caractérisé par la mise en oeuvre des étapes suivantes :
(i) réaction d'un composé de formule II avec un composé de formule III, formules dans lesquelles Q¹, Q² et X¹ répondent à n'importe lesquelles des définitions mentionnées dans la revendication 1, en présence d'une base et en présence d'un catalyseur choisi entre un catalyseur au palladium(0), un catalyseur au palladium(II), un catalyseur au nickel(O) et un catalyseur au nickel(II) ; facultativement en présence d'un initiateur radicalaire ; et facultativement en présence de chlorure de lithium, ce qui donne un composé de formule I ;
(ii) réaction dudit composé de formule I avec un azoture de tri(alkyle en C₁ à C₆)-étain ou un azoture de triphénylétain en présence d'un solvant, puis traitement avec un acide, ce qui donne un composé de formule IX 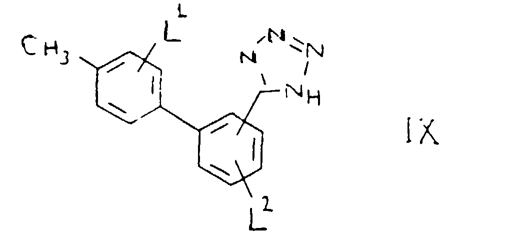
(iii) introduction d'un groupe protecteur dans le noyau tétrazole dudit composé de formule IX, ce qui donne un composé de formule X 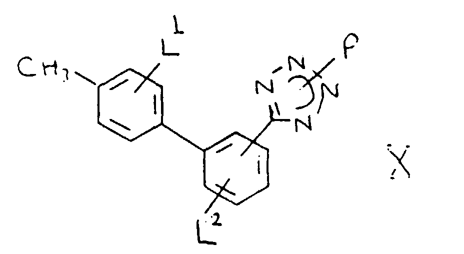 dans laquelle P représente un groupe protecteur ;
(iv) réaction dudit composé de formule X avec un agent de bromation en présence d'un initiateur radicalaire, ce qui donne un composé de formule XI 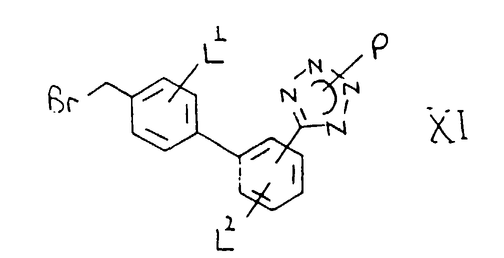
(v) alkylation d'un composé de formule VIII 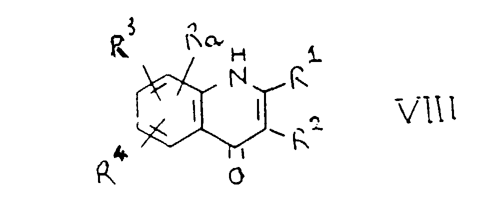 avec ledit composé de formule XI, ce qui donne un composé de formule XII 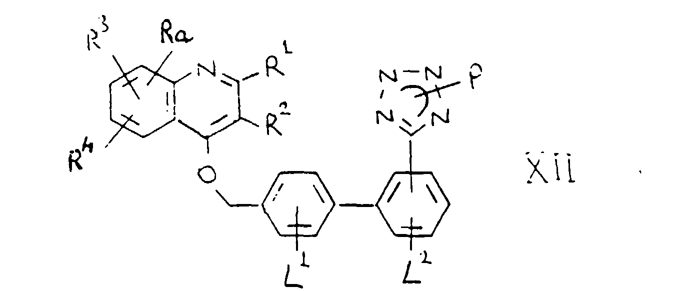 et
(vi) élimination du groupe protecteur P dudit composé de formule XII, ce qui donne ledit composé de formule VI ;
puis : lorsqu'un sel non toxique d'un composé de fromule VI est requis, obtention de ce sel par réaction avec l'acide ou la base approprié donnant un ion physiologiquement acceptable, ou par n'importe quel autre mode opératoire classique de formation de sel ;
R¹, R², R³, R⁴, R⁵, Ra, L¹ et L² répondant à n'importe quelle des définitions précitées.

8. Procédé de production d'un composé de formule VII 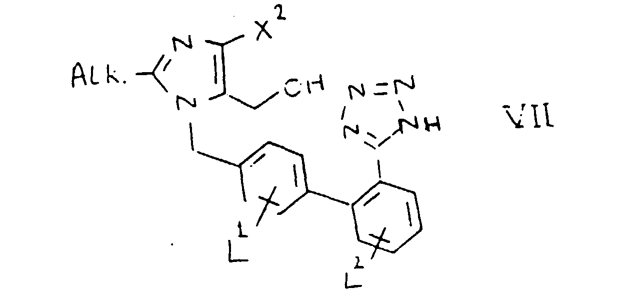 ou d'un de ses sels pharmaceutiquement acceptables, formule dans laquelle Alk représente un groupe alkyle en C₃ à C₁₀ X² est choisi entre l'hydrogène, les groupes fluoro, chloro, bromo, iodo, nitro, trifluorométhyle et cyano ; et L¹ et L² répondent à n'importe lesquelles des définitions mentionnées dans la revendication 1 ou 2, caractérisé par la mise en oeuvre des étapes (i), (ii), (iii) et (iv) définies dans la revendication 7, ce qui donne un composé de formule XI, puis mise en oeuvre des étapes suivantes :
(a) alkylation d'un composé de formule XIII 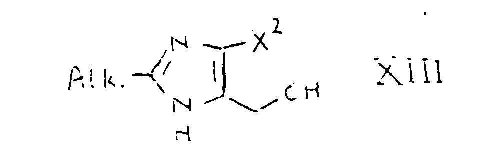 avec ledit composé de formule XI, ce qui donne un composé de formule XIV 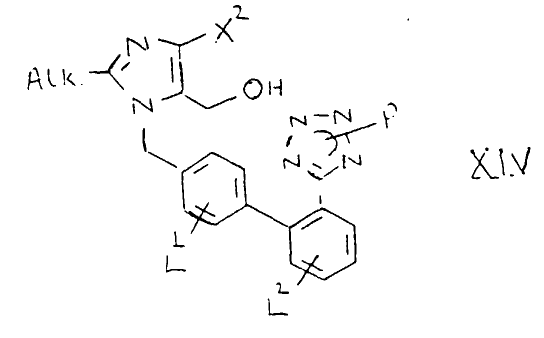 et
(b) élimination du groupe protecteur P dudit composé de formule XIV, ce qui donne ledit composé de formule VII ;
puis : lorsqu'un sel pharmaceutiquement acceptable d'un composé de formule VII est requis, obtention de ce sel par réaction avec l'acide ou la base approprié donnant un ion physiologiquement acceptable, ou par n'importe quel autre mode opératoire classique de formation de sel ; Alk, X², L¹ et L² répondant à n'importe lesquelles des définitions précitées.
